# EUROPEAN PATENT APPLICATION

(11) **EP 3 425 037 A1**
(43) Date of publication of application: **09.01.2019**
(21) Application number: 17180004.8
(22) Date of filing: 06.07.2017
(51) Int. Cl.: C12M 1/26, A61K 35/744, C12M 1/00, A23K 10/10

(54) **MICROBIAL AND FIBROUS BIOMASSES ASSOCIATING DEVICE**

(71) Applicant: TwentyGreen Holding AG, 6039 Root D4 (CH)
(72) Inventor: SUTHERLAND, Duncan, 1027 Lonay (CH); KOLLER, Adrian, 5735 Pfeffikon (CH)
(74) Representative: KATZAROV S.A.

(57) **Abstract**

The invention relates to a probiotic-fiber complexes manufacturing apparatus comprising at least one bioreactor adapted to cultivate and store microorganisms, at least one filtering unit adapted to store fibrous feed material, a tubing and recirculating pump system, and wherein the at least one filtering unit is connected to the at least one bioreactor through the tubing and recirculating pump system so as to provide circulation and isolation of the microorganisms from the bioreactor in the at least one filtering unit, characterized in that the filtering unit is a detachable filtering unit adapted to maintain anaerobic conditions within and comprising a filter adapted to retain the microorganisms in its volume so as to generate Probiotic-fiber complexes.

## Description

### FIELD OF THE INVENTION

The invention relates to the field of industrial food manufacture equipment, more specifically to a filtration/retention vessel capable of associating microbial and fibrous biomasses yielding probiotic-fiber complexes for dietary supplementation.

### BACKGROUND OF THE INVENTION

Dietary supplementation with prebiotic and probiotic products capable of favorably activating intestinal microflora in the intestine has proven beneficial for human and animal health. Prebiotics are food-ingredients that promote the growth of beneficial intestinal microorganisms, e.g. dietary fibers; while probiotics are live microorganisms that, when taken as dietary adjunct, confer health benefits such as improved immune system and metabolic functions. The encapsulation of probiotics with sugars, starch and other typical protective agents provides improved product viability and performance.

One approach shown to protect microbial viability and performance is to associate or load the probiotic microorganisms onto fibrous material to form probiotic-fiber complexes. On the other hand, no efficient scalable industrial processes capable of associating probiotic microorganisms with fibrous feed material are currently available. Creating appropriate and reproducible conditions wherein probiotic microorganisms can homogeneously colonize fiber as well as efficiently digest the fiber at an industrial scale, and that satisfies food industry standards, presents a number of technical challenges.

It is thus an object of the invention to overcome these significant technical problems by providing a filtration/retention vessel that in several ways functions as an artificial and programmable 'herbivorous colon'; an organ wherein the microbiota are known to colonize and degrade dietary fiber. Such an artificial 'herbivorous colon' needs to meet a multitude of technical parameters simultaneously.

An object of the invention is therefore to provide controllable microbial composition.

Another object of the invention is to provide simulation of anaerobic microbial growth conditions in the large intestine where microbes adhere to fiber.

A further object of the invention is to provide capacity to mix biomass, providing microbes with an access to different areas in the fibrous biomass.

Still a further object of the invention is to provide a connect-ability to different bioreactors without cross contaminating, thus providing access of the fibrous material to different classes of microbes.

Another object of the invention is to provide a capacity for fibrous filter material to be co-colonized with microbes isolated from multiple different bioreactors to stimulate symbiotic interactions between different microbes.

Another object of the invention is to provide inlet valves to permit addition of adhesive ingredients such as plasma that can facilitate binding of microbes to fiber.

In this regard, a primary object of the invention is to solve the above-mentioned problems and more particularly to provide an apparatus which fulfills the above listed technical requirements and therefore solves their associated technical problems that is both efficient and scalable for the production of probiotic-fiber complexes as well as prebiotic-fiber complexes for use as dietary supplements.

### SUMMARY ON THE INVENTION

The above problems are solved by the present invention.

A first aspect of the invention is a probiotic-fiber complexes manufacturing apparatus comprising at least one bioreactor adapted to cultivate and store microorganisms, at least one filtering unit adapted to store fibrous feed material, a tubing and recirculating pump system, and wherein the at least one filtering unit is connected to the at least one bioreactor through the tubing and recirculating pump system so as to provide circulation and isolation of the microorganisms from the bioreactor in the at least one filtering unit, characterized in that the filtering unit is a detachable filtering unit adapted to maintain anaerobic conditions within and comprising a filter adapted to retain the microorganisms in its volume so as to generate probiotic-fiber complexes. Thanks to this, one has an artificial "herbivorous colon' wherein the microbiota are known to colonize and degrade dietary fiber in a both efficient and scalable manner for the production of probiotic-fiber complexes as well as prebiotic-fiber complexes for use as dietary supplements

According to a preferred embodiment of the present invention, the at least one filtering unit comprises an inlet percolator providing variable inlet so as to provide microorganisms with an access to different areas of the stored fibrous feed material.

Preferably, the filter comprises fibrous feed material. In this manner, one simplifies its insertion within the filtering unit.

Advantageously, the at least one filtering unit comprises a stirring system adapted to provide mixing of the stored fibrous feed material and the microorganisms. Thus, the colonization is further improved.

According to a preferred embodiment of the present invention, the microorganisms comprise at least one of different types of microbes and different types of yeast. This embodiment is particularly adapted for colonizing the fibrous material with different types of microorganisms.

Preferably, the microorganisms comprise at least one bacteria. Thus, the digestion is further improved.

Advantageously, the bacteria is Archaea. In this manner, the digestion is still further improved.

According to a preferred embodiment of the present invention, the probiotic-fiber complexes manufacturing apparatus comprises at least two bioreactors adapted to cultivate and store different microorganisms from each other and in that the tubing and recirculating pump system provides a separated or filtrated circulation and isolation line for each of the microorganisms connecting the at least one detachable filtering unit to either one of the at least two bioreactors. This embodiment is particularly adapted for mixing different microorganisms within the same filtering unit without contamination.

Preferably, the at least one filtering unit is adapted to be disconnected from the at least one bioreactor when the density of microorganisms within has reached 10⁶-10¹⁴ microorganism/gram fibrous material. Thus, the digestion is optimized.

Advantageously, the at least one filtering unit is adapted be re-connected to different bioreactors containing different microorganisms so as to provide co-colonization of the fibrous feed material. In this manner, the co-colonization is further improved.

According to a preferred embodiment of the present invention, the at least one filtering unit further comprises inlet valves adapted to provide addition of at least one adhesive ingredient that can facilitate binding of microorganisms to the fibrous material. This embodiment is particularly adapted for improving the digestion.

Preferably, at least one adhesive ingredient is comprised in the group consisting of plasma or antibodies. Thus, one can facilitate binding of microbes to the fibers.

Advantageously, the at least one bioreactor comprises gas injection means adapted to inject a H₂-CO₂ gas mix in a 1:4 proportion into the bioreactor. In this manner, anaerobic conditions are properly created.

According to a preferred embodiment of the present invention, the at least one bioreactor comprises a microorganism composition controller adapted to presterilize the microorganisms prior to circulation. This embodiment is particularly adapted for preventing contamination.

### BRIEF DESCRIPTION OF THE DRAWINGS

Further particular advantages and features of the invention will become more apparent from the following non-limitative description of at least one embodiment of the invention which will refer to the accompanying drawings, wherein
- Figure 1 schematically represents a probiotic-fiber complexes manufacturing apparatus according to a preferred embodiment of the present invention;
- Figure 2 schematically represents a preferred embodiment of a detachable filtering unit of the apparatus of the present invention;
- Figure 3 schematically represents a preferred embodiment of a tubing configuration connecting several detachable filtering unit to different bioreactors;
- Figure 4 schematically represents a sterilizable double valve system to be used in the apparatus of the present invention:

### BRIEF DESCRIPTION OF THE INVENTION

The present detailed description is intended to illustrate the invention in a non-limitative manner since any feature of an embodiment may be combined with any other feature of a different embodiment in an advantageous manner.

In the present invention, a detachable filtration/retention vessel 4, 5, 6, also called detachable filtering unit, can be connected to a bioreactor 1, 2, 3 with tubing and recirculating pump system 7, allowing circulation and isolation/retention of microbes from the bioreactor via a novel filtration/retention process. More specifically, the microbial culture is circulated through the detachable filtration/retention vessel 4, 5, 6 and the microbes are trapped within and adhere to a fibrous feed material which may be any one comprised in the group comprising sufficiently finely ground, approved fibrous food or feed material, like algae, grain husk, grass or maize pellets, corn or soybean husks, corn stubble, alfalfa, etc. Preferably, the moisture content in the fibrous substrate reaches saturation due to constant drenching with growth medium, i.e. moisture content > 80%, the temperature is maintained at ideal conditions for target microorganism which, of course, depends on the microorganisms and the entire system (bioreactor, tubing and recirculation system and filtering unit) is pressurized/pressure controlled, for example at 2 bar overpressure.

The vessel can maintain anaerobic conditions such as in the large intestine of mammals and a stirrer/rotation system allows for mixing of the biomass (fibrous material + microoganisms). Once the biomass has sufficiently colonized the filter system and appropriate density is reached, i.e. when the density of microorganisms within has reached 10⁶-10¹⁴ microorganism/gram fibrous material, preferably 10¹² microbes/gram fibrous material, the filtering unit 4, 5, 6 is disconnected from the bioreactor and the biomass can be harvested and integrated into food for human and animal consumption. Alternatively, the the filtering unit 4, 5, 6 can be re-connected to subsequent bioreactors containing different microbes to allow co-colonization of the fibrous feed material with multiple microbial types or multiple strains.

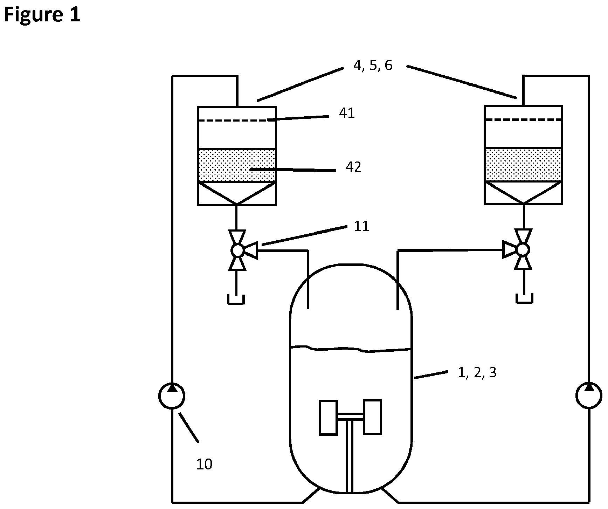

Figure 1 shows an embodiment of the present invention where a probiotic-fiber complexes manufacturing apparatus comprises one bioreactor 1, 2, 3 which is adapted to cultivate and store microorganisms, preferably comprising at least one of different types of microbes such as a bacteria, preferably Archaea, and different types of yeast The apparatus further comprises at least one, here two, detachable filtering units 4, 5, 6 adapted to store fibrous feed material 42 therein, and a tubing and recirculating pump system 7 comprising a recirculating pump 10 for connecting the filtering units v4, 5, 6 to the bioreactor 1, 2, 3 by providing circulation and isolation of the microorganisms from the bioreactor 1, 2, 3 in the filtering units 4, 5, 6.

The filtering unit 4, 5, 6 is a filtering unit adapted to maintain anaerobic conditions within and comprises a filter 42 adapted to retain the microorganisms in its volume so as to generate probiotic-fiber complexes. According to a preferred embodiment, the filter itself may comprise fibrous feed material and it may also be constituted by such fibrous material. The anaerobic conditions are provided by gas injection means adapted to inject a H₂-CO₂ gas mix in a 1:4 proportion into the bioreactor, for example.

Although not represented, the bioreactor 1, 2, 3 may also comprise a microorganism composition controller adapted to presterilize the microorganisms prior to circulation.

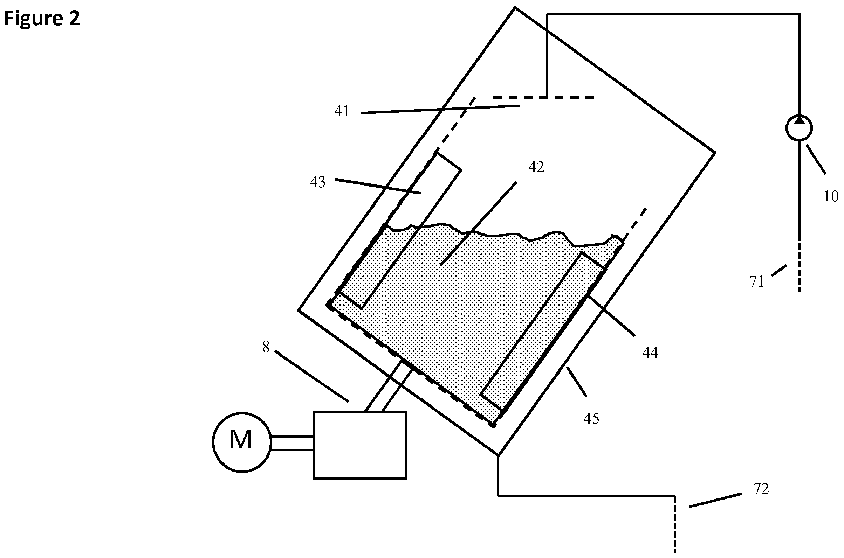

Figure 2 schematically represents a preferred embodiment of a detachable filtering unit of the apparatus of the present invention where the filtering unit 4, 5, 6 comprises an inlet percolator 41 providing a variable inlet so as to provide microorganisms with an access to different areas of the stored fibrous feed material 42'.

Figure 2 also shows a stirring system 8, 44, 45 adapted to provide mixing of the stored fibrous feed material 42' and the microorganisms. The stirring system comprises a low speed drive system 8, a rotating inner hull, preferably a perforated rotating inner hull 44 and a stationary outer hull 45. Preferably, the rotation speed lies in the order of 1 to 3 revolutions per minute, also possible in intervals with a rotation lasting a few seconds followed by a resting period. The motorization pattern of the rotating wall imitates the reticuloruminal motility typical to ruminants. Ideally, the flow rate of supplied growth medium is adapted to the growth stage such that it ensures that the supply of nutrients is not growth limiting.

Avoiding complex engineering solutions for a rotating, sealed shaft into a pressure vessel, the entire filter unit could rotate, but only +/-180 or +/-270°. In this way, hoses/piping can be made flexible, e.g. viton tubing, such that the tubing is wrapped and unwrapped around the rotating filter in an orderly fashion as the motion is executed in a reciprocating fashion. This avoids any rotary feed-through, thereby greatly reducing the complexity and cost of the design.

Although not represented, the at least one filtering unit 4, 5, 6 further comprises inlet valves adapted to provide addition of at least one adhesive ingredient comprised in the group consisting of plasma or antibodies that can facilitate binding of microorganisms to the fibrous material.

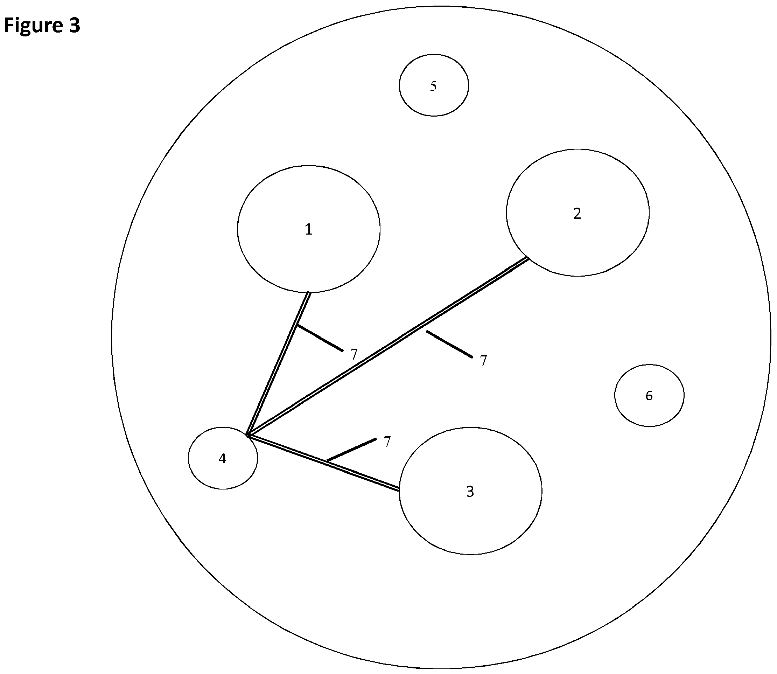

Figure 3 schematically represents a preferred embodiment of a tubing configuration connecting one detachable filtering unit among three available to different bioreactors simultaneously. Indeed, the probiotic-fiber complexes manufacturing apparatus comprises at least two bioreactors 1, 2, 3 adapted to cultivate and store different microorganisms from each other and the tubing and recirculating pump system 7 provides a separated or filtrated circulation and isolation line for each of the microorganisms connecting the at least one detachable filtering unit 4, 5, 6 to either one of the at least two bioreactors 1, 2, 3.

As is represented, one can see that the Detachable Filtering Unit (DFU) 1 is connectable to multiple bioreactors 1, 2, 3 so as to be re-connected to different bioreactors 1, 2, 3 containing different microorganisms so as to provide co-colonization of the fibrous feed material, either sequentially or simultaneously via the tubing and recirculating system 7.

More particularly, in the schematic example of this figure, DFU1 is connected simultaneously to bioreactors 1, 2, 3. Connectability of the DFU to multiple bioreactors enables the colonization of products from bioreactors 1, 2, 3 within the same filtering unit. Products from bioreactors 1, 2, 3 may, for example, contain microorganisms that when collocalized on fibers (probiotic-fibre complex) have the potential to interact symbiotically resulting in a synergistic probiotic effect.

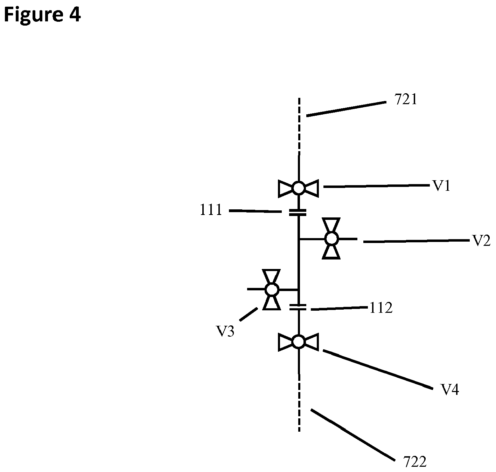

Figure 4 schematically represents a sterilizable double valve system which is installed on the return or drain line 72 of the tubing and recirculating system 7.

This system comprises an upstream valve V1, a clean steam inlet valve V2 a clean steam/condensate exit valve V3, a downstream valve V4, a first quick connection 111 located between the upstream valve V1 and the clean steam inlet valve V2 and a second quick connection 112 located between the clean steam/condensate exit valve V3 and downstream valve V4.

This system is ideally located on the return or drain line 72 so as to divide it into an upstream process line 721 which is connecting the system to the filtering unit and a downstream process line 722 which is connecting the system to the reactor

This sterilization system works as follows.

First, all valves V1, V2, V3, V4 are closed. Once this is the case, the first and second quick connection, preferably tri-clamps, are mated and thereafter, a steam clean inlet is connected to V2.

At this moment, V3 is opened, V2 is also slightly opened, simultaneously or slightly after, to let the steam sterilize the conduits from V1 downstream side to V4 upstream side for 20 to 30 minutes.

Finally, one closes all valves and lets temperature equilibrates and once this is achieved, one opens V1 and V4 to enable process connection.

The design of the sterilizable double valve must ensure the drain point at V3 is the lowest point in the conduits such that no condensation may accumulate in any pipe section between V1 and V4.

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This, for example, is particularly the case regarding the different apparatuses which can be used.

### Reference list

- 1, 2, 3:: Bioreactor
- 4, 5, 6:: Detachable filter units
- 7:: Tubing and recirculating pump system
- 8:: Low speed drive system
- 10:: Recirculating pump
- 11:: Return or drain valve
- 41:: Inlet percolator
- 42:: Fibrous substrate filter cartridge
- 42':: Tumbling fibrous filter substrate
- 43:: Catch paddle
- 44:: Perforated rotating inner hull
- 45:: Stationary outer hull
- 71:: Supply line
- 72:: Return or drain line
- 721:: Upstream process line, e.g. connection to filter unit
- 722:: Downstream process line, e.g. return to reactor
- 111:: First quick connection
- 112:: Second quick connection
- V1:: Upstream valve
- V2:: Clean steam inlet valve
- V3:: Clean steam/condensate exit valve
- V4:: Downstream valve

## Claims

1. Probiotic-fiber complexes manufacturing apparatus comprising
at least one bioreactor (1, 2, 3) adapted to cultivate and store microorganisms,
at least one filtering unit (4, 5, 6) adapted to store fibrous feed material (42, 42'),
a tubing and recirculating pump system (7), and
wherein the at least one filtering unit (4, 5, 6) is connected to the at least one bioreactor (1, 2, 3) through the tubing and recirculating pump system (7) so as to provide circulation and isolation of the microorganisms from the bioreactor (1, 2, 3) in the at least one filtering unit (4, 5, 6),
**characterized in that** the filtering unit (4, 5, 6) is a detachable filtering unit adapted to maintain anaerobic conditions within and comprising a filter (42) adapted to retain the microorganisms in its volume so as to generate probiotic-fiber complexes.

2. Probiotic-fiber complexes manufacturing apparatus according to claim 1, **characterized in that** the at least one filtering unit (4, 5, 6) comprises an inlet percolator (41) providing variable inlet so as to provide microorganisms with an access to different areas of the stored fibrous feed material (42, 42').

3. Probiotic-fiber complexes manufacturing apparatus according to claim 1 or 2, **characterized in that** the filter (42) comprises fibrous feed material.

4. Probiotic-fiber complexes manufacturing apparatus according to any one of claims 1 to 3, **characterized in that** the at least one filtering unit (4, 5, 6) comprises a stirring system (8, 44, 45) adapted to provide mixing of the stored fibrous feed material (42') and the microorganisms.

5. Probiotic-fiber complexes manufacturing apparatus according to any one of claims 1 to 4, **characterized in that** the microorganisms comprises at least one of different types of microbes and different types of yeast.

6. Probiotic-fiber complexes manufacturing apparatus according to claim 5, **characterized in that** the microorganisms comprises at least one bacteria.

7. Probiotic-fiber complexes manufacturing apparatus according to claim 6, **characterized in that** the bacteria is Archaea.

8. Probiotic-fiber complexes manufacturing apparatus according to any one of claims 1 to 7, **characterized in that** it comprises at least two bioreactors (1, 2, 3) adapted to cultivate and store different microorganisms from each other and **in that** the tubing and recirculating pump system (7) provides a separated or filtrated circulation and isolation line for each of the microorganisms connecting the at least one detachable filtering unit (4, 5, 6) to either one of the at least two bioreactors (1, 2, 3).

9. Probiotic-fiber complexes manufacturing apparatus according to any one of claims 1 to 6, **characterized in that** the at least one filtering unit (4, 5, 6) is adapted to be disconnected from the at least one bioreactor (1, 2, 3) when the density of microorganisms within has reached 10⁶-10¹⁴ microorganism/gram fibrous material.

10. Probiotic-fiber complexes manufacturing apparatus according to claim 9, **characterized in that** the at least one filtering unit (4, 5, 6) is adapted be re-connected to different bioreactors (1, 2, 3) containing different microorganisms so as to provide co-colonization of the fibrous feed material.

11. Probiotic-fiber complexes manufacturing apparatus according to any one of claims 1 to 10, **characterized in that** the at least one filtering unit (4, 5, 6) further comprises inlet valves adapted to provide addition of at least one adhesive ingredient that can facilitate binding of microorganisms to the fibrous material.

12. Probiotic-fiber complexes manufacturing apparatus according to claim 11, **characterized in that** the at least one adhesive ingredient is comprised in the group consisting of plasma or antibodies.

13. Probiotic-fiber complexes manufacturing apparatus according to any one of claims 1 to 12, **characterized in that** the at least one bioreactor (1, 2, 3) comprises gas injection means adapted to inject a H₂-CO₂ gas mix in a 1:4 proportion into the bioreactor.

14. Probiotic-fiber complexes manufacturing apparatus according to any one of claims 1 to 13, **characterized in that** the at least one bioreactor (1, 2, 3) comprises a microorganism composition controller adapted to presterilize the microorganisms prior to circulation.
